# EUROPÄISCHE PATENTSCHRIFT

(11) **EP 4 389 012 B1**
(45) Veröffentlichungstag und Bekanntmachung des Hinweises auf die Patenterteilung: **04.02.2026**
(21) Anmeldenummer: 22215513.7
(22) Anmeldetag: 21.12.2022
(51) Int. Cl.: A61B 6/10, A61B 6/00

(54) **TAKTILES SENSORCOVER UND HERSTELLUNGSVERFAHREN UND MEDIZINISCHES GERÄT**
TACTILE SENSOR COVER AND MANUFACTURING METHOD AND MEDICAL DEVICE
JOINT DE CAPTEUR TACTILE ET PROCÉDÉ DE FABRICATION ET DISPOSITIF MÉDICAL

(43) Veröffentlichungstag der Anmeldung: 26.06.2024
(73) Patentinhaber: Siemens Healthineers AG, 91301 Forchheim (DE)
(72) Erfinder: Schmidt, Verena, 92681 Erbendorf (DE)
(74) Vertreter: Siemens Healthineers Patent Attorneys

(56) Entgegenhaltungen:
- EP-A1- 3 447 462
- DE-A1- 102013 222 115
- DE-A1- 102013 225 575
- YUANYUAN XU ET AL: "The Boom in 3D-Printed Sensor Technology", SENSORS, vol. 17, no. 5, 1 May 2017 (2017-05-01), CH, pages 1166, XP055683486, ISSN: 1424-8220, DOI: 10.3390/s17051166
- WANG ZHEN ET AL: ""Safe Skin" - A Low-Cost Capacitive Proximity-Force-Fusion Sensor for Safety in Robots", 2021 IEEE/RSJ INTERNATIONAL CONFERENCE ON INTELLIGENT ROBOTS AND SYSTEMS (IROS), IEEE, 27 September 2021 (2021-09-27), pages 807 - 813, XP034051681, DOI: 10.1109/IROS51168.2021.9636308

## Beschreibung

Unabhängig vom grammatikalischen Geschlecht eines bestimmten Begriffes sind Personen mit männlicher, weiblicher oder anderer Geschlechteridentität mit umfasst.

Die Erfindung betrifft ein taktiles Sensorcover zur Detektion einer Kollision mit einem mehrschichtigen, flächigen Aufbau, ein Herstellungsverfahren für dasselbe sowie ein medizinisches Gerät umfassend das taktile Sensorcover.

Medizinische Bildgebungsanlagen wie z.B. einem Angiographiegerät oder einer Fluoroskopieanlage, aber auch anderes medizinisches Gerät, werden während eines Untersuchungs- oder Behandlungsverlaufs bewegt. Die Bewegung kann dabei ein Verstellen einzelner Komponenten der Anlage, umfassen. Bspw. wird ein C-Bogen eines Angiographie-Geräts zum Erfassen von dreidimensionaler Bildinformation über einen interessierenden Körperbereich eines Patienten geschwenkt. Daneben kann auch die gesamte medizinische Bildgebungsanlage bewegt werden, bspw. wenn es sich um ein mobiles Gerät handelt, welches für eine Untersuchung zunächst in eine Untersuchungsumgebung gebracht werden muss. Die Bewegung einer medizinischen Bildgebungsanlage kann manuell gesteuert, autonom oder teilautonom erfolgen. Dabei gilt immer das Erfordernis, durch die Bewegung der Bildgebungsanlage weder Mensch noch Gerät in der direkten Umgebung der Anlage zu gefährden. Auch Personen und Geräte in der Umgebung der Anlage können sich bewegen. Daher werden heutzutage sichere Taktilsensoren für den Kollisionsschutz verwendet. Taktilsensoren sind an der medizinischen Bildgebungsanlage angeordnet und erfassen eine Berührung bzw. eine Kollision der Anlage mit einer Person bzw. einem Gegenstand der Umgebung. Basierend auf dem Sensorsignal können dann verschiedene Sicherheitsmaßnahmen aktiviert werden, bspw. ein Bewegungsstopp oder ein Warnsignal für einen Nutzer der Anlage. In weiteren Anwendungen werden Taktilsensoren eingesetzt, um Bildgebungskomponenten, bspw. eine Röntgenstrahlenquelle, möglichst nahe an einen Patienten anzufahren, um eine bessere Bildgebung zu erzielen. In der Zukunft wird eine Kollisionsüberwachung bzw. ein Kollisionsschutz auf Grund des vermehrten Einsatzes eines autonomen Bewegungsbetriebs für medizinische Bildgebungsanlagen noch wichtiger. Besonders boden- oder deckengebundene, mobile Systeme sollen sich zukünftig mehr und mehr autonom bewegen.

Ein Kollisionsschutz kann taktile, eine Berührung erfassende, aber auch berührungslose Sensoren umfassen, die bereits eine Annäherung der bewegten Anlage an einen Gegenstand der Umgebung anzeigen. Berührungslosen Sensoren zur Kollisionserkennung ist gemein, dass sie einen Totbereich direkt vor dem Sensor aufweisen, in dem eine Annäherung nicht erfasst werden kann. Eine Absicherung des Bereichs direkt vor dem Sensor ist somit nicht für alle Anwendungen bzw. nicht in allen Situationen möglich. Bspw. kann eine an einer mit einer Bildgebungsanlage in Form eines Magnetresonanztomographen interagierende Patientenliege angeordnete Kollisionssensorik nach korrekter Positionierung an der Gantry für die Bilddatenerfassung abgeschaltet werden. In diesem Zustand wird ein Objekt in direkte räumliche Nähe vor der Kollisionssensorik positioniert. Bei Wiedereinschalten der Kollisionssensorik wird dieses Objekt nicht erfasst. Aus dieser Einschränkung resultiert auch, dass es mittels berührungsloser Sensorik schwerer ist, einen sicheren Kollisionsschutz zu realisieren. Hierzu wird dann neben dem berührungslosen Sensor ein weiterer sicherer taktiler Kollisionssensor vorgesehen. Entsprechend ist die Auslegung bzw. Marktverfügbarkeit von standardisierten, sicheren Kollisionssensoren erschwert.

Kapazitive Sensoren als spezielle Ausführungsvariante für eine berührungslose Nahfeldsensorik eignen sich nur bedingt für eine Nutzung auf mobilen Plattformen, wie sie insbesondere für mobile medizinische Bildgebungsanlagen bekannt sind.

Kapazitive Kollisionssensoren gibt es in geerdeter und ungeerdeter Ausführung. Geerdete kapazitive Sensoren haben eine Detektionsreichweite von bis zu 10 cm, sollten dafür jedoch an einer definierten Position im Raum fixiert, also idealerweise selbst unbewegt sein. Ungeerdete kapazitive Sensoren habe hingegen nur eine sehr eingeschränkte Detektionsreichweite von ca. 1 cm bis 2 cm und können daher nur einen sehr eingeschränkten Sicherheitsbereich um die medizinische Bildgebungsanlage realisieren, welcher für viele medizinischen Anwendungen zu gering ist. Eine weitere Herausforderung bei kapazitiven Sensoren beim Einsatz im medizinischen Umfeld ist, dass diese für ein optimales Ergebnis am Einsatzort kalibriert werden müssen. Wechselnde Umgebungen wie zusätzliche medizinische Geräte, Infusionsständer und/oder unterschiedliche Tischanbauten können das Kalibrierergebnis beeinflussen., was Damit einher geht ein hoher Konzeptionierungs- bzw. Serviceeinsatz einhergeht, denn letztlich sieht die Umgebung jeder Anlage anders aus. Im Zweifel sorgt eine kapazitive Kollisionssensorik für allzu häufige Bewegungsstopps, sodass Untersuchungsabläufe gestört und verzögert werden.

Taktile Kollisionssensoren sind bspw. in Form von in das Gehäuse (außen weiche Schicht, innen härtere Schicht) integrierten miniaturisierten Schaltleisten oder in Form von lackierten taktilen Gehäuseteilen bekannt, bei denen bei Kollision durch Verformen einer die Außenseite bildenden Weichcoverschicht ein elektrischer Kontakt zu einer innenliegenden Hartcoverschicht unter Überbrückung einer dazwischen liegenden Abstandsschicht geschlossen oder ein Widerstand verändert wird. Diese Lösungen sind aus den folgenden Gründen derzeit sehr teuer:
Die Verbindung bzw. das Fügen von Hart- und Weichcoverteilen ist aufwändig. Beide Teile werden wegen der verschiedenen Härteanforderungen getrennt voneinander gefertigt und müssen im Anschluss gefügt werden. Dadurch ergeben sich unerwünschte Maßabweichungen und Formveränderungen, bspw. durch einen an die Fertigung anschließenden Trocknungs-, Schrumpfungs- oder Härtungsprozess. Entsprechend müssen die Einzelteile oft manuell und aufwändig nachbearbeitet werden. Dieser Effekt wirkt sich umso stärker aus, je größer die Coverteile sind. Zudem können optisch unschöne bzw. aus Hygiene-Sicht nachteilhafte Kanten und Fugen zwischen den einzelnen Coverteilen bestehen bleiben. Eine Nachbearbeitung der gesamten Coverteil-Oberflächen, bspw. ein Schleifen oder ein Lackieren, kann ebenfalls zur Einhaltung von Hygiene-Standards erforderlich sein.

Im Bereich der Fluoroskopie werden bislang auch taktile Kollisionssensoren aus Textil eingesetzt. Diese unterliegen jedoch im Hinblick auf ihre dreidimensionale Formbarkeit und ihre Oberflächenqualität starken Einschränkungen. Durch die Verwendung eines Textils als Sensorträgermaterial sind Textilsensoren kaum formstabil. Zudem ist eine textile Oberflächenbeschaffenheit typischerweise strukturiert bzw. uneben und daher schmutzanfällig und schwer reinigbar. Im Vergleich zu den Taktilsensoren mit härteren Coverteilen weisen die textilen Taktilsensoren zudem eine größere Aufbauhöhe auf, was ggf. den Footprint der Anlage nachteilig beeinflusst.

### Die Publikation "The Boom in 3D-Printed Sensor

Technology" (YUANYUAN XU ET AL, SENSORS, Bd. 17, Nr. 5, 1. Mai 2017 (2017-05-01), Seite 1166, XP055683486, CH ISSN: 1424-8220, DOI: 10.3390/s17051166) bietet einen allgemeinen Überblick über die Entwicklung und Anwendung von 3D-gedruckten Sensoren. Es beschreibt verschiedene 3D-Drucktechniken wie Fused Deposition Modeling (FDM), Direct Ink Writing (DIW) und Stereolithografie (SLA) und deren Einsatz in der Sensorherstellung. Die Anwendungen umfassen eine breite Palette von Sensoren, darunter Kraft-, Druck-, Dehnungs-, akustische und optische Sensoren.

Die Druckschrift EP 3 447 462 A1 beschreibt eine Schutzeinrichtung umfassend wenigstens einen flexiblen, zumindest auf einen Teil der Oberfläche des mit der Schutzeinrichtung auszustattenden Bauteils formangepasst aufbringbaren, elastisch komprimierbaren Sicherheitsüberzug mit Kollisionserfassung und eine mit der Kollisionserfassung verbundene Auswerteeinheit auf.

Demgegenüber ist es Aufgabe der vorliegenden Erfindung, Taktilsensoren bereitzustellen, die im Hinblick auf ihre Maßhaltigkeit, den Fertigungsaufwand und die Herstellungskosten optimiert sind.

Diese Aufgabe wird gelöst durch ein taktiles Sensorcover zur Detektion einer Kollision sowie ein Herstellungsverfahren für dasselbe gemäß unabhängigen Ansprüchen. Die Aufgabe wird auch gelöst durch ein medizinisches Gerät umfassend das taktile Sensorcover gemäß einem weiteren unabhängigen Anspruch. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die Erfindung ist in einem ersten Aspekt gerichtet auf ein taktiles Sensorcover zur Detektion einer Kollision. Das taktile Sensorcover weist einen mehrschichtigen, flächigen Aufbau auf. Das bedeutet, das Sensorcover besteht aus mehreren Einzelschichten und weist in zumindest einer Raumrichtung einen deutlich geringeren, insbesondere flacheren Aufbau auf als in den beiden übrigen Raumrichtungen. Das Cover muss dabei jedoch nicht flächig in einer Ebene ausgebildet sein, sondern es kann eine beliebige dreidimensionale Freiform einnehmen. Mit anderen Worten können die einzelnen Schichten des Sensorcovers in Ausführungen eben, also im Wesentlichen zweidimensional ausgebildet sein (unter Vernachlässigung der Aufbauhöhe), oder die einzelnen Schichten können gekrümmt ausgebildet sein, also einen dreidimensionalen Verlauf zeigen. Insofern weist auch jede einzelne der Schichten des Sensorcovers in zumindest einer Raumrichtung einen deutlich geringeren, insbesondere flacheren Aufbau auf als in den beiden übrigen Raumrichtungen.

In Ausführungen können wenigstens zwei Schichten dieselbe Dicke/Höhe aufweisen. In anderen Ausführungen können alle Schichten dieselbe Höhe aufweisen. In weiteren Ausführungen können alle Schichten des Sensorcovers eine voneinander unterschiedliche Dicke aufweisen. Es sind erfindungsgemäß auch Ausführungen denkbar, in denen wenigstens eine Schicht über ihre Fläche hinweg eine veränderliche Höhe aufweist, also eine Schicht nicht an jeder Stelle gleich dick ist.

Das Sensorcover kann folglich eine Freiform aufweisen, bei der alle Schichten gleich dick sind. Das Sensorcover kann alternativ eine Freiform aufweisen, bei der verschiedene Schichten unterschiedlich dick ausgebildet sind. Damit lässt sich räumlich aufgelöst eine unterschiedliche Drucksensitivität des Sensorcovers realisieren. An einigen Stellen, wird folglich eine größere äußere Kraft benötigt, um den Sensorauszulösen als an anderen Stellen des Sensorcovers.

Der schichtartige Aufbau des Sensorcovers wird gebildet durch
- eine reversibel deformierbare, eine Außenseite des Sensorcovers bildende Deckschicht,
- eine starre, eine Innenseite des Sensorcovers bildende Grundschicht,
- eine zwischen Deckschicht und Grundschicht verlaufende Sensoreinheit umfassend
   o zwei Sensorschichten, und
- eine zwischen den beiden Sensorschichten angeordnete, reversibel kompressible Abstandsschicht.

Deck- und Grundschicht bilden folglich eine äußere Begrenzung für das Sensorcover. In Ausführungen können weitere Schichten vorgesehen sein. Bspw. kann auf der der Sensorschicht gegenüberliegenden Außenseite der Grundschicht eine weitere Abstands- oder Pufferschicht vorgesehen sein. Zwischen genannten Schichten können ebenfalls zusätzliche Schichten umfasst sein.

Das taktile Sensorcover zeichnet sich dadurch aus, dass wenigstens zwei Schichten als mittels einer Technik der additiven Fertigung gebildete Schichten ausgebildet sind. Die additive Fertigung beschreibt und umfasst Fertigungsverfahren, bei denen dreidimensionale Werkstücke schichtartig aus einem Ausgangsmaterial bzw. einem Werkstoff, ggf. umfassend verschiedene Materialien hergestellt werden. Hierbei können verschiedene Ausgangsmaterialien sowie verschiedene Methoden der additiven Fertigung, bspw. der 3D-Druck, zum Einsatz kommen. Die additive Fertigung bietet große Vorteile im Hinblick auf die Flexibilität der Formgebung der gefertigten Werkstücke, denn die additive Fertigung kommt ohne Anwendung von Guss- oder Pressformen oder Negative für das Werkstück aus. Ferner können Werkstücke mittels additiver Fertigung besonders maßhaltig hergestellt werden. Im Bezug auf die einsetzbaren Ausgangsmaterialien sind inzwischen viele Varianten verfügbar.

Vorteil des erfindungsgemäßen Sensorcovers ist, dass wenigsten zwei Schichten des Schichtaufbaus mittels eines additiven Fertigungsverfahrens erzeugt werden. Besonders vorteilhaft werden alle Schichten mittels eines additiven Fertigungsverfahrens hergestellt. So kommen die Vorteile der Herstellung für alle Schichten des Sensorcovers zum Tragen. Insofern nutzt das erfindungsgemäße Sensorcover die Vorteile der additiven Fertigungstechnologie.

Das taktile Sensorcover ist dazu ausgebildet, eine Berührung mit einem Objekt, einem Gegenstand oder einer Person in der Umgebung des Sensorcovers zu erfassen. Zu diesem Zweck umfasst das Sensorcover eine Sensoreinheit, die ausgebildet ist, eine Berührung und damit einhergehende Kompression des Sensorcovers zu detektieren und in Abhängigkeit der Berührung ein Sensorsignal zu erzeugen. Die Sensoreinheit umfasst vorliegend zwei Sensorschichten, die ausgebildet sind, zur Erzeugung des Sensorsignals zusammenzuwirken.

In bevorzugter Ausführung sind die Sensorschichten daher elektrisch leitfähig ausgebildet und umfassen ein metallisches Material. Mit anderen Worten können die erste (benachbart zur Deckschicht) und die zweite (benachbart zur Grundschicht) Sensorschicht aus einer flächigen Metallschicht gebildet sein, bspw. Kupfer. In einer anderen Ausführung können die erste und die zweite Sensorschicht aus einem Trägermaterial, bspw. ein Kunststoff, insbesondere ein Thermoplast gebildet sein, in welchem Metallpartikel umfasst sind. Mit aktivierter Sensoreinheit werden die Sensorschichten mit einer Spannung beaufschlagt. Wird die Deckschicht und zumindest teilweise auch die erste Sensorschicht durch eine Kollision mit einem Objekt deformiert und die Abstandsschicht derart komprimiert, dass sich die Sensorschichten berühren, fließt zwischen den beiden Sensorschichten ein Strom. Dieser Strom ist repräsentativ für die Kollision. In dieser Ausführung wirken die Sensorschichten wie ein Schaltelement.

In anderen Ausführungen des taktilen Sensorcovers kann auch die zwischen den Sensorschichten verlaufende Abstandsschicht leitfähig ausgebildet sein, bspw. durch Zusatz eines metallischen Pulvers zu deren Ausgangsmaterial. In dieser Ausführung werden die Sensorschichten ebenfalls mit Spannung beaufschlagt. Durch Kompression der Abstandsschicht im Falle einer Kollision ändert sich durch die Variation des Abstands zwischen den Sensorschichten messbar der ohmsche Widerstand. Dieser ist repräsentativ für einen auf das Sensorcover wirkenden Druck und kann als entsprechendes Sensorsignal aufgegeben werden.

Wie eingangs erläutert, sind die Sensorschichten erfindungsgemäß leitfähig ausgebildet. Insofern müssen an den Sensorschichten Anschlusselemente zum Anlegen einer Spannung bzw. zum Messen eines Stromes zwischen den Sensorschichten vorgesehen werden.

Gemäß der Erfindung umfassen die Sensorschichten jeweils ein einstückig angeformtes elektrisches Anschlusselement. Das Anschlusselement wird also direkt zusammen mit den Sensorschichten erzeugt, sodass ein nachträgliches Verbinden des Anschlusselements und der Sensorschicht entfallen kann. Erfindungsgemäß ist das Anschlusselement zusammen mit einer der Sensorschichten durch metallisches Aufdampfen, Metallsintern oder eine andere Technik der additiven Fertigung hergestellt. Insbesondere sind Sensorschicht und Anschlusselement in dieser Ausführung aus demselben Material ausgebildet. In Ausführungen des Sensorcovers ragt ein Anschlusselement über die Grundfläche der Sensorschicht hinaus, bspw. erstreckt sich das Anschlusselement als schmale, flache Zuleitung senkrecht von einer Seite der Sensorschicht weg. Hierbei können Grund- und Deckschicht in Ausführungen über die Grundfläche der Sensorschicht hinausragen und somit eine Träger- bzw. Auflagefläche für das Anschlusselement bilden, an welche das Anschlusselement bei der Fertigung direkt angefügt werden kann. Die Anschlusselemente können in Ausführungen auch eine Stütz- bzw. Trägerschicht umfassen, um die Sensorschichten ausreichend zu stabilisieren, bspw. dann, wenn die Sensorschichten und damit auch die Anschlusselemente aus einer dünnen Metallschicht gebildet sind. Dabei kann das Anschlusselement dieselbe Aufbauhöhe wie die Sensorschicht aufweisen oder flacher ausgebildet sein. In weiteren Ausführungen kann das Anschlusselement auch eine dickere Aufbauhöhe als die Sensorschicht aufweisen. Insbesondere dann, wenn eine spezielle Anschlussschnittstelle nachgebaut werden muss, kann das Anschlusselement höher als eine Sensorschicht ausgebildet sein. Derart können bspw. bekannte oder standardisierte Stecker zur Kontaktierung der Sensorschicht eingesetzt werden, aber auch komplexe Anschlussgeometrien realisiert werden. Bspw. können an sich bekannte Klinkenstecker zur Verbindung der Sensorschichten eingesetzt werden. Das Anschlusselement kann zumindest an dem von der Sensorschicht entfernten Ende eine spezifische Verbindungs- bzw. Kontaktgeometrie aufweisen, bspw. kann das Anschlusselement insgesamt eher flach ausgebildet sein, an seinem Ende aber ein rundes Kontaktierungselement aufweisen. Sensorschicht und Anschlusselement liegen in Ausführungen ausschließlich in einer gemeinsamen Ebene. In weiteren Ausführungen kann das Anschlusselement auch eine dreidimensionale Form aufweisen, In einer Variante kann das Anschlusselement bspw. zunächst senkrecht seitlich aus der Sensorschicht geführt werden und in einem bestimmten Abstand zur Sensorschicht bspw. in einem 90°-Winkel nach oben oder unten geführt werden. Sowohl das Anschlusselement als auch die Sensorschicht können also dreidimensionale Formen annehmen, also gekrümmt ausgebildet sein. Insbesondere kann derart auch das Anschlusselement an die Bauform des Sensorcovers angepasst sein.

In wieder anderen Ausführungen kann das Anschlusselement als ein einstückig an eine Sensorschicht angefügtes Kontaktloch oder einstückig angefügter Kontaktdraht ausgebildet sein. Das Kontaktloch ist dabei als Loch oder Ausnehmung in der Sensorschicht ausgebildet, wobei es sich in Ausführungen senkrecht nach oben oder unten durch mindestens eine der angrenzenden Schichten, insbesondere die Grundschicht, erstrecken kann. In das Kontaktloch kann dann ein Kontaktstift eingeführt werden, um die Sensorschicht zu kontaktieren. Auch ein Kontaktdraht zeigt erfindungsgemäß einen Verlauf nach oben oder unten durch weitere Schichten des Sensorcovers hindurch. Kontaktloch und/oder Kontaktdraht sind vorteilhaft von eine Isolationsschicht umgeben, die das Anschlusselement jeweils von den übrigen Schichten abschirmt. Derart ermöglicht der Aufbau des Sensorcovers vorteilhaft eine in Bezug auf das Sensorcover innenliegende Verkabelung, sodass der Footprint des Sensorcovers vorteilhaft beibehalten werden kann.

In Ausführungen des Sensorcovers, in denen mehrere Sensorsegmente umfasst sind, die weiter unten noch beschrieben werden, können verschiedene Bauformen des Anschlusselements in einem Sensorcover zum Einsatz kommen.

Besondere Vorteile ergeben sich, wenn die wenigstens zwei mittels additiver Fertigung gebildeten Schichten des Sensorcovers als im Rahmen der additiven Fertigung gefügte Schichten ausgebildet sind. Mit anderen Worten umfasst das Sensorcover nicht nur mehrere Schichten, die einzeln und unabhängig voneinander mittels einer additiven Fertigungstechnik hergestellt und anschließend miteinander verbunden werden. Sondern wenigstens zwei Schichten innerhalb des Schichtaufbaus des Sensorcovers werden im Verlauf der Fertigung beider Schichten direkt miteinander verbunden. Derart kann zum einen wenigstens ein nachgeschalteter Fügeschritt eliminiert werden. Zudem kann auf bspw. chemische Fügemittel verzichtet werden. Zum anderen können derart Fugen und Unebenheiten in der Oberfläche des Sensorcovers vorteilhaft reduziert oder sogar vermieden werden. Aufwändige manuelle Nachbearbeitungsschritte zur Oberflächenoptimierung oder zur Herstellung einer erforderlichen Maßhaltigkeit der jeweiligen Schicht, wie Schleifen oder Fräsen, können entfallen. Insbesondere können die miteinander verbundenen Schichten dasselbe Material umfassen, aber auch aus verschiedenen Materialien aufgebaut sein. Zu beachten ist eine geeignete Materialwahl für derart gefügte, benachbarte Schichten. Hier kommen selbstredend nur diejenigen Materialien mit entsprechender Kompatibilität bzw. ausreichenden Hafteigenschaften in Frage.

Besonders bevorzugt werden alle Schichten des Sensorcovers mittels einer Technik der additiven Fertigung erzeugt und direkt bei ihrer Herstellung mit einer anderen der Schichten verbunden.

Wenigstens eine, bevorzugt mehrere Schichten, sind in Ausführungen der Erfindung aus einem Material umfassend einen Kunststoff gebildet. Besonders die Deckschicht, die Grundschicht und/oder die Abstandsschicht sind entsprechend ausgebildet und damit prädestiniert für eine Fertigung mittels einer additiven Technik.

Kunststoffe lassen sich unterteilen in Duroplaste, Thermoplaste und Elastomere und bestehen aus Polymeren, also Makromolekülen. Die molekulare Struktur sowie die makroskopischen Eigenschaften von Kunststoffen lassen sich in weiten Grenzen durch das Herstellungsverfahren und die Beimischung von Additiven variieren. Da Duroplaste sich durch eine feste Struktur und lange Formbeständigkeit auszeichnen, kommen sie bspw. für die Herstellung der starren Grundschicht in Frage, die im Sensorcover zur Aufbringung einer Gegenkraft zur in das Sensorcover eingebrachten Kompressionskraft dient. Die Grundschicht ist erfindungsgemäß starr, also i.W. unflexibel ausgebildet und übernimmt auch die Funktion einer Tragstruktur. Insofern kann die Grundschicht bspw. auf Epoxidharz-Basis erzeugt werden.

Thermoplastische Kunststoffe zeigen auch eine grundsätzlich feste Struktur, die sich jedoch durch Einbringen von Wärme reversibel verändern lässt. Durch bestimmtes Strukturieren von Schichten des Sensorcovers oder eine Variation der Materialstärke oder Materialdichte lassen diese Materialien auch eine reversible Verformbarkeit zu. Damit eignen sich Thermoplaste insbesondere als Materialien zur Herstellung von Grundschicht, Abstandsschicht und/oder Deckschicht. Typische mögliche Thermoplaste sind bspw. Polyethylen, Polyamid, Polyethylenephthalat, Polystyrol oder Polyvenylchlorid.

Elastomere sind per se elastisch und daher als Ausgangsmaterial für die Abstandsschicht oder die Deckschicht geeignet, da beide Schichten bei einer Kompression reversibel verformt und oder kompressibel ausgebildet sein müssen, um die Funktion des taktilen Sensorcovers zu unterstützen. Bspw. kann Kautschuk zur Herstellung der Abstandsschicht und/oder der Deckschicht verwendet werden.

Insbesondere werden zur Herstellung der Schichten röntgentransparente und röntgenbeständige Ausgangsstoffe gewählt. Das bedeutet, dass die Schichten des Sensorcovers nicht bzw. kaum Signale bei einer Röntgenbildgebung verursachen bzw. dass die Schichten durch Röntgenstrahlung in ihren mechanischen Eigenschaften nicht beeinflusst werden. Dies garantiert eine Langlebigkeit des Sensorcovers und vermeidet unnötige Bildartefakte durch das taktile Sensorcover bei Einsatz in einem medizinischen Gerät in Form einer medizinischen Röntgenbildgebungs- oder Röntgenstrahlentherapieanlage, die mittels Röntgenstrahlung medizinische Bilddaten erzeugt oder krankhaftes Gewebe eines Patienten behandelt.

Die Vorteile der additiven Fertigung zur Herstellung des Sensorcovers werden besonders deutlich, wenn die mittels der additiven Fertigung zusammengefügten Schichten aus demselben Ausgangsmaterial hergestellt sind. Selbst wenn sich die einzelnen Schichten in ihrer Stärke und ihrer Struktur unterscheiden, geht die Erfindung davon aus, dass das Sensorcover dadurch langfristig besonders formstabil ist. Zudem kann ein Produktionsprozess, wie er weiter unten noch genauer beschrieben wird, besonders einfach und schnell ablaufen, je weniger verschiedene Ressourcen eingesetzt werden. Insbesondere geht die Erfindung davon aus, dass bei Herstellung mehrerer Sensorcoverschichten aus ein und demselben Material nur eine Fertigungsmaschine benötigt wird, was eine Effektivierung und Kostenreduktion für den Fertigungsprozess bedeutet.

In bevorzugter Ausführung sind insbesondere die Deckschicht und die Grundschicht aus demselben Material gebildet. Dabei wird besonders bevorzugt, wie eingangs schon erläutert, die mechanische Stabilität der beiden Schichten über die Dichte des Materials bestimmt. Mechanische Eigenschaften wie elastische Verformbarkeit oder Steifigkeit können in diesen Ausführungen des Sensorcovers allein über eine Variation der Dichte des Materials und/oder über eine Variation der Materialstärke eingestellt werden. Insbesondere kann in Ausführungen mit demselben Material und bei gleicher Materialdichte die Deckschicht eine geringere Aufbauhöhe aufweisen wie die Grundschicht, um die Deckschicht reversibel verformbar auszubilden und die Grundschicht starr auszubilden. In anderen Ausführungen kann bei unabhängig von einer geeigneten Aufbauhöhe der beiden Schichten für die Grundschicht ein Vollmaterial verarbeitet werden, während für die zumindest in gewissen Grenzen verformbare Deckschicht Luft- oder Gaseinschlüsse oder Poren oder dergleichen vorgesehen sind, um die Materialdichte zu reduzieren.

Neben der Deckschicht muss auch die Abstandsschicht reversibel deformierbar, insbesondere reversibel kompressibel bzw. komprimierbar ausgebildet sein, um verlässlich und wiederholt eine Kollision mit einem Objekt detektieren zu können. In Ausführungen des taktilen Sensorcovers ist die Abstandsschicht daher als Textilgewirk, Netzstruktur, Gitterstruktur oder Schaumstruktur ausgebildet bzw. weist diese auf. Alternativ dazu umfasst die Abstandsschicht eine Vielzahl von sich zwischen den Sensorschichten erstreckenden Stützbalken.

Alle die genannten Strukturen weisen einen dreidimensionalen lockeren Aufbau umfassend Lücken, Hohlräume und/oder Löcher auf. Diese sorgen einerseits dafür, dass sich im Falle einer Kompression ein Kontakt zwischen erster und zweiter Sensorschicht schließt bzw. bewirken sie eine größere mechanische Verformbarkeit des Materials.

In besonders bevorzugten Ausführungen ist das taktile Sensorcover nicht nur ausgebildet, eine Kollision mit einem Objekt zu erfassen, sondern auch zu detektieren, wo in Bezug auf die Fläche des Sensorcovers eine Berührung stattgefunden hat. Mit anderen Worten ist das Sensorcover in Ausführungen ausgebildet, eine Kollision räumlich aufgelöst zu erfassen. Zu diesem Zweck weisen die beiden Sensorschichten jeweils wenigsten zwei Sensorsegmente auf. Sprich, die Grundfläche der beiden Sensorschichten ist unterteilt bzw. segmentiert. Dabei ist jeweils ein erstes Sensorsegment der ersten Sensorschicht mit einem ersten Sensorsegment der zweiten Sensorschicht deckungsgleich angeordnet. Entsprechend wirken jeweils erste und jeweils zweite Sensorsegmente als eigenständige taktile Sensoreinheiten. Über die Anzahl der Sensorsegmente einer Sensorschicht lässt sich die örtliche Auflösung weiter erhöhen. Insbesondere können Sensorcover in Bereichen größere Sensorsegmente und dementsprechend dort eine geringe örtliche Auflösung aufweisen. In anderen Bereichen können die Sensorsegmente hingegen kleiner ausgebildet sein und eine entsprechend hohe Ortsauflösung bereitstellen. Die Form der einzelnen Sensorsegmente kann ebenfalls applikationsspezifisch variieren.

Selbstredend weist jedes Sensorsegment ein wie oben bereits beschriebenes eigenes Anschlusselement auf, wobei jeweils die Anschlusselemente von den ersten, zweiten, etc. Sensorsegmenten der ersten und zweiten Sensorschichten miteinander verbunden sind. Hierbei können insbesondere randständige Sensorsegmente senkrecht seitlich austretende Anschlusselemente aufweisen und zentral bzw. mittig platzierte Sensorsegmente sich direkt aus der Sensorschicht nach oben oder unten durch weitere Schichten des Sensorcovers erstreckende Kontaktlöcher als Anschlusselemente umfassen.

Um die Sensoreinheit vor äußeren Störeinflüssen, insbesondere Feuchtigkeit bzw. Flüssigkeiten zu schützen, umfasst das taktile Sensorcover in einer besonders bevorzugten Ausführung eine in sich geschlossene Schutzkapsel, die sich schichtartig zwischen Deckschicht und erster Sensorschicht, Grundschicht und zweiter Sensorschicht und jeweils an allen Seiten der Sensorschichten sowie der Abstandsschicht erstreckt. Die Schutzkapsel bildet also einen Container bzw. eine Schutzhülle für die Sensoreinheit und trennt räumlich die Komponenten der Sensoreinheit, insbesondere die Sensorschichten, von den übrigen Teilen des Sensorcovers ab. Die Wände der Schutzkapsel sind ebenfalls schichtartig und flächig aufgebaut und folgen der Formgebung der übrigen Schichten des Sensorcovers. Die Wände der Schutzkapsel sind in diesem Sinne ebenfalls als Schichten des erfindungsgemäßen Sensorcovers zu verstehen. Sie können aus den oben beschriebenen Materialien bestehen, müssen die wie oben beschriebenen mechanischen Eigenschaften aufweisen und können insbesondere auch mittels einer Technik der additiven Fertigung hergestellt werden. insbesondere können auch die Wände der Schutzkapsel direkt bei der Fertigung mit wenigstens einer benachbarten Schicht des Sensorcovers gefügt werden und die oben beschriebenen Vorteile nutzen.

Die Schutzkapsel weist in Ausführungen des Sensorcovers in den Seitenwänden und/oder in ihren Deck-/Grundwänden Öffnungen auf, durch die die Anschlusselemente der Sensorschichten nach außen geführt werden.

Die vorliegende Erfindung betrifft in einem zweiten Aspekt ein medizinisches Gerät umfassend ein erfindungsgemäßes taktiles Sensorcover.

Das medizinische Gerät ist gekennzeichnet durch einen Einsatz in einer medizinischen Umgebung sowie die Tatsache, dass es zumindest Komponenten umfasst, die bewegbar ausgebildet sind oder welches vollständig bewegbar ausgebildet ist und insofern einer Kollisionsüberwachung bedarf.

Ein medizinisches Gerät kann eine Anlage zur medizinischen Bildgebung, wie ein Computertomograph, eine Angiographie-Anlage, ein klassisches Durchleuchtungsröntgengerät, ein Magnetresonanztomograph, ein molekulare Bildgebungsanlage, ein Ultraschallgerät oder dergleichen sein. In weiteren Ausführungen kann das medizinische Gerät eine therapeutische Anlage sein, bspw. eine Strahlentherapieanlage oder dergleichen. In weiteren Ausführungen kann es sich bei dem medizinischen Gerät auch um ein Assistenzgerät, insbesondere ein robotisches Assistenzgerät, handeln. Umfasst können hier bspw. sein: Assistenz-Roboter, mobile EKG-Geräte, Strahlenschutzvorrichtungen oder dergleichen.

Wie eingangs bereits erläutert, kann das taktile Sensorcover insbesondere wegen der Fertigung mittels einer Technik der additiven Fertigung, in eine beliebige dreidimensionale Form gebracht werden. Insofern weisen in bevorzugter Ausführung des medizinischen Geräts die Schichten des taktilen Sensorcovers eine dreidimensionale Freiform entsprechend einer Gehäuseform des medizinischen Geräts auf. Mit anderen Worten wird die dreidimensionale Form des Sensorcovers einer Gehäuseform des medizinischen Geräts angeformt bzw. nachgebildet. Das taktile Sensorcover ersetzt folglich wenigstens ein Gehäuseteil des medizinischen Geräts. Bspw. ersetzt das Sensorcover das Gehäuse eines an einer C-Bogen-Anlage angeordneten Röntgenstrahlers oder des gleichfalls an der C-Bogen-Anlage angeordneten Röntgendetektors. Auch der C-Bogen selbst kann mit einem daran angeformten erfindungsgemäßen Sensorcover ummantelt sein. Ersetzt das taktile Sensorcover ein Gehäuseteil eines medizinisches Geräts, bildet seine Deckschicht die Außenseite des Gehäuses des medizinischen Geräts.

Ein nächster Aspekt der vorliegenden Erfindung ist auf ein Verfahren zur Herstellung eines taktilen Sensorcovers gerichtet. Das Sensorcover umfasst dabei, wie eingangs erläutert,
- eine reversibel deformierbare, eine Außenseite des Sensorcovers bildende Deckschicht,
- eine starre, eine Innenseite des Sensorcovers bildende Grundschicht,
- eine zwischen Deckschicht und Grundschicht verlaufende Sensoreinheit umfassend
   o zwei Sensorschichten, und
- eine zwischen den beiden Sensorschichten angeordnete, reversibel kompressible Abstandsschicht.

Das Verfahren umfasst eine Vielzahl von Schritten. Ein erster Schritt ist gerichtet auf das Fertigen der reversibel kompressiblen Abstandsschicht mittels einer Technik der additiven Fertigung. Ein zweiter Schritt ist gerichtet auf ein Aufbringen der ersten und zweiten Sensorschicht jeweils auf den beiden gegenüberliegenden Außenseiten der Abstandsschicht. Das Aufbringen umfasst in Ausführungen sowohl das Herstellen der Sensorschichten als auch das Fügen der Sensorschichten an die Abstandsschicht. Ein weiterer Schritt ist gerichtet auf ein Aufbringen von Deckschicht und Grundschicht jeweils mittels einer Technik der additiven Fertigung auf die Außenseiten der beiden Sensorschichten. Auch im dritten Schritt kann das Aufbringen auch ein Herstellen/Fertigen der Deck- und Grundschicht umfassen.

In diesem Aspekt wird das erfindungsgemäße Sensorcover also von innen nach außen aufgebaut. Gemäß diesem Aspekt kann die Abstandsschicht in dem ersten Schritt insbesondere als Free-Foam Teil ausgebildet sein, welches zunächst aus einem Material dreidimensional gedruckt und anschließend unter Erhitzung expandiert wird.

In einem weiteren Aspekt betrifft die Erfindung ein alternatives Verfahren, zur Herstellung eines wie oben beschriebenen Sensorcovers. In diesem Aspekt wird das Sensorcover ausgehend von Grund- oder Deckschicht Schicht für Schicht aufgebaut. Die Aufbaurichtung ist hierbei variabel und bspw. durch das gewählte Herstellungsverfahren vorgegeben. In einem ersten Schritt erfolgt also bspw. zunächst die Fertigung der Grundschicht mittels einer Technik der additiven Fertigung. In einem zweiten Schritt wird dann die zweite Sensorschicht auf die obere Außenseite der Grundschicht aufgebracht. In einem dritten Schritt erfolgt ein Aufbringen der reversibel kompressiblen Abstandsschicht mittels einer Technik der additiven Fertigung auf die zweite Sensorschicht. In einem vierten Schritt wird die erste Sensorschicht auf die obere Außenseite der Abstandsschicht aufgebracht. In einem letzten Schritt wird die Deckschicht mittels einer Technik der additiven Fertigung auf die Außenseite der ersten Sensorschicht aufgebracht.

Umfasst das Sensorcover eine Schutzkapsel, die weiter oben bereits beschrieben wurde, umfasst ein Herstellungsverfahren eines Sensorcovers an jeweils geeigneter Stelle einen weiteren Schritt, in welchem die Schutzkapsel mittels einer Technik der additiven Fertigung hergestellt und in bevorzugter Ausführung mit wenigstens einer benachbarten Schicht gleichzeitig gefügt wird.

Für jede einzelne Schicht des Sensorcovers kann in Ausführungen der Erfindung grundsätzlich eine beliebige Technik der additiven Fertigung eingesetzt werden. Ein Schritt eines erfindungsgemäßen Herstellungsverfahrens umfasst folglich eine der folgenden Techniken der additiven Fertigung: 3D-Druck, Lasersintern, Schmelzschichten, Fused Filament Verfahren oder ein Multi Jet Modeling.

Bevorzugt werden mehrere, besonders bevorzugt alle Schichten des Sensorcovers mit bzw. in ein und derselben Maschine hergestellt, ohne das Bauteil zwischenzeitlich entnehmen zu müssen. Derart kann das Sensorcover durch den Herstellungsprozess im Hinblick auf Maßhaltigkeit und Passgenauigkeit optimiert werden.

Insbesondere wenn Anschlusselemente in Form von Kontaktlöchern oder Kontaktdrähten vorgesehen sind, können die Schritt auch miteinander verschachtelt bzw. einer intermittierenden Reihenfolge ausgeführt werden. Es kann auch vorgesehen werden, dass einzelne Schichten teilweise gefertigt werden, während oder zwischen der Fertigung oder dem Fügen weiterer Schichten.

In einer weiteren Ausführung des erfindungsgemäßen Verfahrens zur Herstellung eines taktilen Sensorcovers umfasst das Aufbringen der ersten und oder der zweiten Sensorschicht auf eine der benachbarten Schichten ein metallisches Sintern, ein Aufdampfen eines Metalls, ein Auflegen einer Metallschicht oder eine einer Technik der additiven Fertigung mit einem Metallpartikel umfassenden Kunststoff umfasst. Eine weitere Alternative zur Fertigung der Sensorschichten stellt das Ecocoating dar. Die einzelnen Techniken sind an sich bekannt und werden daher nicht näher erläutert.

Da die Sensorschichten leitfähig ausgebildet sind und zu deren Herstellung ein Metall verarbeitet werden muss, können in den oben genannten zweiten bzw. vierten Schritten andere als Methoden der additiven Fertigung eingesetzt werden. Insbesondere das Aufdampfen einer dünnen Metallschicht auf eine Außenseite einer benachbarten Schicht stellt eine einfache Möglichkeit zur Erzeugung einer Sensorschicht dar.

In weiteren Ausführungen des erfindungsgemäßen Verfahrens können einzelne Schichten zunächst bspw. mittels additiver Technik vorgefertigt und dann miteinander verbunden werden. Bspw. können die Deckschicht und die Grundschicht erfindungsgemäß auch einzeln gefertigt werden. Dasselbe gilt für die Sensorschichten. Die Sensorschichten können bspw. dann auf die Deck- und die Grundschicht aufgelegt werden. Durch Fertigen und Anfügen der Abstandsschicht können dann Deck-, Sensorschicht und Abstandsschicht miteinander verbunden werden. Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine Querschnittsansicht eines taktilen Sensorcovers in einem Ausführungsbeispiel der Erfindung,
- FIG 2: eine Querschnittsansicht eines taktilen Sensorcovers in einem anderen Ausführungsbeispiel der Erfindung,
- FIG 3: eine Draufsicht des taktilen Sensorcovers gemäß Figur 1,
- FIG 4: eine Detailansicht eines taktilen Sensorcovers in einem anderen Ausführungsbeispiel der Erfindung,
- FIG 5: eine Detailansicht eines taktilen Sensorcovers in einem weiteren Ausführungsbeispiel der Erfindung,
- FIG 6: eine Querschnittsansicht eines taktilen Sensorcovers in einem weiteren Ausführungsbeispiel,
- FIG 7: eine Ansicht eines medizinischen Geräts gemäß einem Ausführungsbeispiel der vorliegenden Erfindung,
- FIG 8: eine schematische Darstellung eines erfindungsgemäßen Verfahrens gemäß einem Ausführungsbeispiel der vorliegenden Erfindung, und
- FIG 9: eine schematische Darstellung eines erfindungsgemäßen Verfahrens gemäß einem anderen Ausführungsbeispiel der vorliegenden Erfindung.

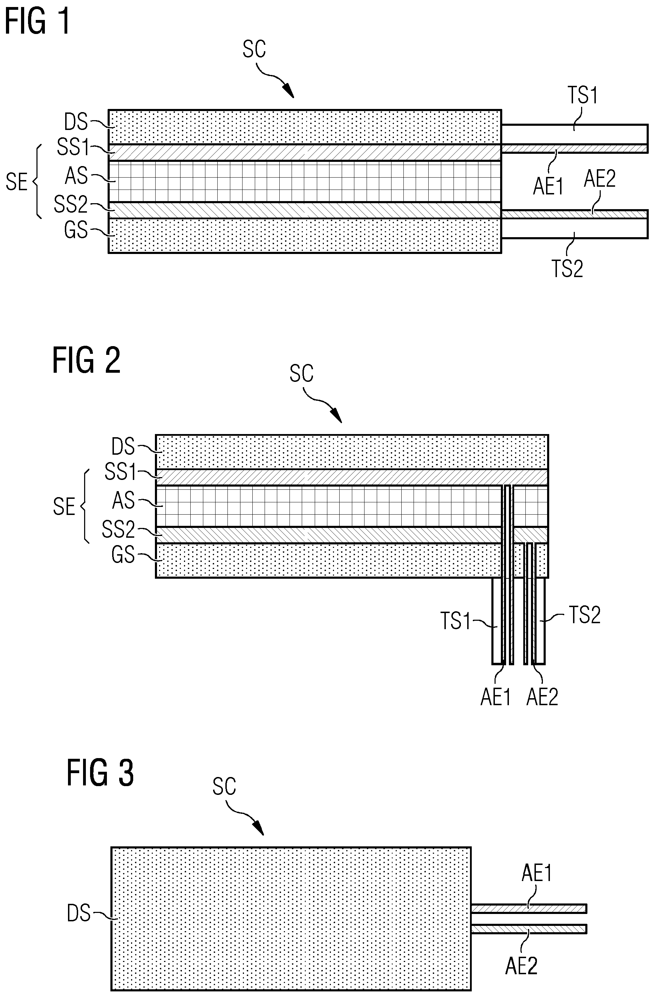

**Figur** 1 zeigt eine Querschnittsansicht eines taktilen Sensorcovers SC in einem Ausführungsbeispiel der Erfindung.

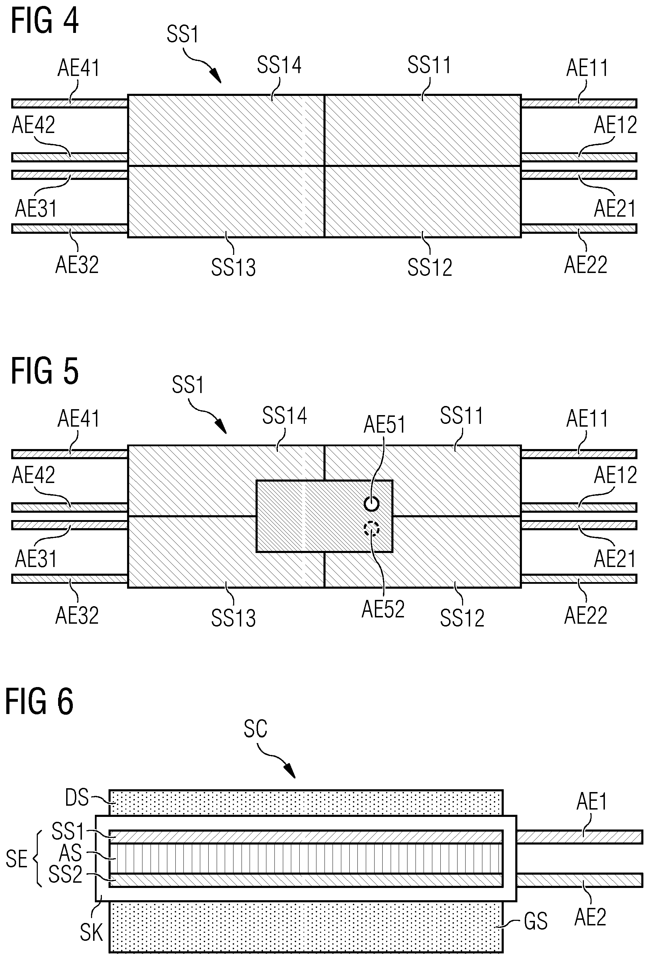

Das taktile Sensorcover SC dient der Detektion einer Kollision mit einem Objekt in der Umgebung. Dieses kann ein ortsfestes oder ein mobiles Objekt, bspw. ein Mensch oder ein anderes medizinisches Gerät sein. Das Sensorcover SC weist einen mehrschichtigen, flächigen Aufbau auf. Das Sensorcover umfasst eine reversibel deformierbare, eine Außenseite des Sensorcovers bildende Deckschicht DS. Diese ist im montierten Zustand des Sensorcovers Sc nach außen gerichtet und bildet die Außenseite des Geräts, an welchem das Sensorcover SC montiert ist, bspw. ein medizinisches Gerät 1 wie in Figur 5 dargestellt. Die Deckschicht DS ist flexibel bzw. verformbar und damit ausgebildet, eine von außen einwirkende Kraft oder ein von außen einwirkenden Druck auf innere Schichten des Sensorcovers zu übertragen.

Das Sensorcover umfasst weiter eine starre, eine Innenseite des Sensorcovers bildende Grundschicht GS. Diese ist i.W. unbeweglich und inkompressibel ausgebildet und baut im Kollisionsfall eine Gegenkraft zu der von außen einwirkenden Kraft auf.

Die eine Kollision detektierende Sensoreinheit SE umfasst eine zwischen Deckschicht DS und Grundschicht GS verlaufende erste und zweite Sensorschicht SS1, SS2. Zwischen beiden Sensorschichten SS1, SS2 verläuft eine reversibel kompressible Abstandsschicht AS. Mit Verformung der Deckschicht DS im Falle einer Kompression verformt sich auch die erste Sensorschicht SS1 sowie die Abstandsschicht AS. Diese wird komprimiert.

Die Sensorschichten SS1, SS2 sind beide leitfähig ausgebildet und umfassen ein metallisches Material. In dieser Ausführung umfassen die Sensorschichten SS1, SS2 dünne, metallische, gleichmäßig über die Grundfläche der Sensorschichten SS1, SS2 verteilte Leiterbahnen, die in ein Trägermaterial der Sensorschichten SS1, SS2 eingelegt sind. Um die Sensorschichten SS1, SS2 besonders leicht elektrisch kontaktieren zu können, umfassen die Sensorschichten SS1, SS2 jeweils ein einstückig angeformtes elektrisches Anschlusselement AE1, AE2 welche jeweils aus den Sensorschichten herausragen. Die Anschlusselemente sind genau wie die Flächen der Sensorschichten aufgebaut und umschließen bspw. genau eine metallische Leiterbahn. Die Anschlusselemente AE1, AE2 umfassen vorliegend jeweils eine separate erste und zweite Trägerschicht TS1, TS2, um eine ausreichende mechanische Stabilität der Anschlusselemente AE1, AE2 zu gewährleisten. Über die Anschlusselemente AE1, AE2 werden die Sensorschichten SS1, DD2 mit einer Spannung beaufschlagt. Die Anschlusselemente AE1, AE2 sind in **Figur 3** in einer Draufsicht gezeigt.

In der vorliegenden Ausführung ist die Abstandsschicht AS aus einem porösen, schaumartigen Material ausgebildet. Wird die Abstandsschicht AS so sehr zusammengedrückt, dass sich die beiden Sensorschichten SS1, SS2 berühren, fließt wegen der Poren bzw. Hohlräume in der Abstandsschicht ein Strom zwischen den beiden Sensorschichten SS1, SS2. Dieser Strom zeigt die Kollision an. Die Sensoreinheit SE ist ausgebildet, ein entsprechendes Sensorsignal zu erzeugen und auszugeben.

Erfindungsgemäß sind wenigstens zwei Schichten des Sensorcovers SC als mittels einer Technik der additiven Fertigung gebildete Schichten ausgebildet. Insbesondere die Deckschicht, Grundschicht und Abstandsschicht DS, GS, AS und ggf. weitere Schichten wie bspw. die Schutzkapsel SK (vgl. Figur 6) kommen hierfür in Frage, denn hierfür können Materialien ohne spezielle Beimischungen wie bspw. Metalle, verwendet werden. Materialien für die genannten Schichten können aus den verschiedenen für unterschiedliche Anwendungen geeignete Kunststoffen gewählt werden. Die Ausführung des Sensorcovers SC gemäß Figur 1 weist die erfindungsgemäße Besonderheit auf, dass die wenigstens zwei mittels additiver Fertigung, bspw. 3D-Druck, gebildeten Schichten DS, GS, AS als im Rahmen der additiven Fertigung gefügte Schichten ausgebildet sind. Das heißt, wenigstens eine der Schichten, bevorzugt alle oder mehrere Schichten, wird, während sie hergestellt wird, auch gleich mit einer ihrer Nachbarschichten verbunden. Derart werden Aufwand und Kosten der Produktion reduziert. Zudem kann wegen des Einsatzes eines additiven Fertigungsverfahrens auch die Maßhaltigkeit der einzelnen Schichten und damit des gesamten Sensorcovers SC verbessert werden, um optischen als auch hygienischen Erfordernissen zu entsprechen.

Wie bereits erläutert, unterscheiden sich die mechanischen Eigenschaften der verschiedenen Schichten des Sensorcovers SC. Dennoch ist es aus produktionstechnischer Sicht ein großer Vorteil, wenn für die Herstellung eines Sensorcovers SC so wenig Ausgangsstoffe eingesetzt werden wie möglich. Insofern weist das in Figur 1 gezeigte Sensorcover SC eine Deckschicht DS und eine Grundschicht GS auf, die aus demselben Material gebildet sind und bei denen die mechanische Stabilität allein über die Dichte des Materials bestimmt bzw. festgelegt ist. So bestehen in dieser Ausführung beide Schichten DS, GS bspw. aus Polyethylen, welches mit einer geeigneten Methode der additiven Fertigung in die passende Struktur und Form gebracht wird. Da die Deckschicht DS im Vergleich zur Grundschicht GS weicher bzw. deformierbar ausgebildet sein muss, ist die Dichte des Polyethylens in der Deckschicht DS geringer als in der Grundschicht GS.

Wie eingangs erläutert, ist insbesondere auch die Abstandsschicht AS dadurch gekennzeichnet, dass sie elastisch kompressibel ist. Der komprimierte Zustand ist also wiederholbar herstellbar. Um diese mechanische Eigenschaft gut darzustellen und gleichzeitig die Berührung von erster und zweiter Sensorschicht SS1, SS2 zu erlauben, ist die Abstandsschicht AS als Textilgewirk ausgebildet. Sprich, die Abstandsschicht umfasst ein textiles Gewirk, welches eine lockere, Luftkammern aufweisende Struktur zeigt. Alternativ kann die Abstandsschicht als ein mittels 3D-Druck erzeugte Bauteil aus Kunststoff ausgebildet sein, welches einem textilen Gewirk nachgebildet ist. Unter Krafteinwirkung lässt es sich zusammenpressen und geht anschließend wieder in seine ursprüngliche Form zurück. Alternativ könnte die Abstandsschicht aus einer aus einem Kunststoff gebildeten Netzstruktur, Gitterstruktur oder Schaumstruktur ausgebildet sein, die allesamt die mechanischen Vorgaben erfüllen.

Das in **Figur 2** gezeigte Sensorcover SC unterscheidet sich von dem in Figur 1 gezeigten Sensorcover SC durch Anschlusselemente AE1, AE2, die hier in Form von nach unten geführten Kontaktlöchern ausgebildet sind, die sich in vertikaler Richtung nach unten durch die Abstandsschicht AS, die zweite Sensorschicht SS2 und die Grundschicht GS erstrecken. Unterhalb der Grundschicht weisen die Anschlusselemente AE1, AE2 wieder Trägerschichten TS1, TS2 auf, die sich in dieser Ausführung ebenfalls vertikal erstrecken. Im Inneren der Kontaktlöcher ist ein Hohlraum, der bspw. direkt zur Kontaktierung der Sensorschichten genutzt werden kann, indem ein passender Stecker in die Kontaktlöcher eingeführt wird. Anstelle der Kontaktlöcher könnten in an sich analoger Weise Kontaktdrähte vorgesehen sein. Nicht gezeigt werden in Figur 2 Isolationsschichten, die mantelartig um die Kontaktlöcher angeordnet sind und die Anschlusselemente AE1, AE2 von den durchsetzten Schichten des Sensorcovers SC trennen.

**Figur 4** zeigt eine Detailansicht eines taktilen Sensorcovers SC in einem anderen Ausführungsbeispiel der Erfindung.

Das hier gezeigte Sensorcover SC unterscheidet sich von dem in den Figuren 1 und 2 gezeigten Varianten dadurch, dass es eine Kollision mit einem Objekt in der Umgebung ortsaufgelöst detektieren kann. Das taktile Sensorcover SC umfasst hier Sensorschichten mit Sensorsegmenten. Gezeigt ist die Sensorschicht SS1, die vier gleichgroße, rechteckige Sensorsegmente SS11, SS12, SS13, SS14 aufweist. Die Segmente der Sensorschicht SS2 würden eine analoge Form und Anordnung zeigen und sind jeweils von der Sensorschicht SS1 verdeckt. Jedes Sensorsegment SS11, SS12, SS13, SS14 weist analog zu den Segmenten der zweiten Sensorschicht SS2 ein Anschlusselement AE11, AE21, AE31, AE41 auf, welches jeweils mit den Anschlusselementen AE12, AE22, AE32, AE42 zusammenwirkt. Derart bilden die erste und die zweite Sensorschicht SS1, SS2 in dieser Ausführung vier einzelne Kollisionssensoren der Sensoreinheit SE aus, die unabhängig voneinander eine Kollision erfassen können. Deckschicht DS, Abstandsschicht AS und Grundschicht GS können in dieser Ausführung wie die von Figur 1 ausgebildet sein. Insbesondere sind diese Schichten geschlossen über alle Sensorsegmente hinweg ausgebildet.

**Figur 5** zeigt eine Detailansicht eines taktilen Sensorcovers SC in einem weiteren Ausführungsbeispiel der Erfindung. Hier umfasst die erste Sensorschicht SS1 im Vergleich zu Figur 4 ein weiteres Sensorsegment SS15, welches mittig bzw. zentral zwischen den übrigen vier Sensorsegmenten angeordnet ist. Die übrigen Sensorsegmente SS11 bis SS14 weisen nun keine rechteckige Grundform mehr auf. Dies zeigt, dass die Sensorsegmente eine beliebige Grundform annehmen können. Die zweite Sensorschicht SS2 weist auch hier eine zur ersten Sensorschicht SS1 analoge Unterteilung in Segmente auf. Da das Sensorsegment SS15 innerhalb der ersten Sensorschicht SS1 zentral angeordnet ist, ist ein Anschlusselement AE51 in Form eines Kontaktlochs vorgesehen, welches sich in Bezug auf die Schichten senkrecht erstreckt. Das Kontaktloch kann bspw. wie mit Bezug zu Figur 2 beschrieben, ausgebildet sein. Ein in Deckung mit dem Sensorsegment SS15 angeordnetes Sensorsegment der zweiten Sensorschicht SS2 weist entsprechend ein weiteres als Kontaktloch ausgebildetes Anschlusselement AE51 auf.

**Figur 6** zeigt eine Querschnittsansicht eines taktilen Sensorcovers SC in einem weiteren Ausführungsbeispiel.

Die Abstandsschicht AS dieser Ausführung ist zum einen gebildet aus einer Vielzahl von sich zwischen den Sensorschichten SS1, SS2 erstreckenden Stützbalken. Zum anderen ist in dieser Ausführung auch die Abstandsschicht AS leitfähig, bspw. durch Beimischung von Metallpulver, ausgebildet, sodass sich durch eine Kompression der Abstandsschicht AS im Falle einer Kollision der Widerstand zwischen den Sensorschichten SS1, SS2 verringert. Derart können auch schon leichte Berührungen des Sensorcovers SC mit der Sensoreinheit SE wahrgenommen und gemeldet werden. Die Sensorschichten SS1, SS2 sind in dieser Ausführung als flächige Metallschichten ausgebildet, die bspw. beidseitig auf die Abstandsschicht AS nach deren Herstellung aufgedampft wurden. Alternativ wurden die Sensorschichten SS1, SS2 auf die Außenseiten der Deck- bzw. Grundschicht DS, GS, aufgedampft. Auch wenn in der schematischen Ansicht nicht dargestellt, können auch die Anschlusselemente AE1, AE2 jeweils auf einer der Schichten mit aufgedampft werden und einstückig mit den Sensorschichten SS1, SS2 verbunden sein.

Auch in dieser Ausführung sind Deck- und Grundschicht DS, GS vorteilhaft aus demselben Material, hier bspw. Polyurethan, aufgebaut. Schematisch und nicht maßstäblich ist hier veranschaulicht, dass die Deformierbarkeit der Deckschicht DS über eine geringere Schichtdicke ggü. der stabilen Grundschicht im Rahmen der Fertigung eingestellt wurde.

Die hiesige Variante des taktilen Sensorcovers SC umfasst zudem eine in sich geschlossene Schutzkapsel SK, die sich schichtartig zwischen Deckschicht DS und erster Sensorschicht SS1, Grundschicht GS und zweiter Sensorschicht SS2 und jeweils an allen Seiten der Sensorschichten SS1, SS2 sowie der Abstandsschicht AS erstreckt. Die Schutzschicht wird vorzugsweise aus einem Vollmaterial, aber auch mittels einer Technik der additiven Fertigung, erzeugt. Die Schutzkapsel weist bevorzugt keine Poren, Öffnungen oder dergleichen auf und bildet so eine Schutzschicht für die eingeschlossene Sensoreinheit SE. Insbesondere Flüssigkeiten können so von der Sensoreinheit SE ferngehalten werden, was sich positiv auf die Lebensdauer des Sensorcovers SC auswirkt. Je nach Materialwahl für die Schutzkapsel SK kann diese auch einen Schutz vor Schnitte mit scharfen Gegenständen, bspw. einem Skalpell oder ähnlichem bieten. Selbstredend lassen sich sämtliche Vorteile der Erfindung, die sich aus der Anwendung einer Technik der additiven Fertigung ergeben, auch auf die Schutzkapsel SK übertragen. Die Schutzkapsel SK weist vorliegend Öffnungen bzw. Auslässe für den Anschlusselemente AR1, AE1 oder ggf. mehr als zwei, auf. Idealerweise liegen die Ränder der Öffnungen lückenlos um die Anschlusselemente, sodass auch an diesen Stellen ein Eindringen einer Flüssigkeit verhindert wird.

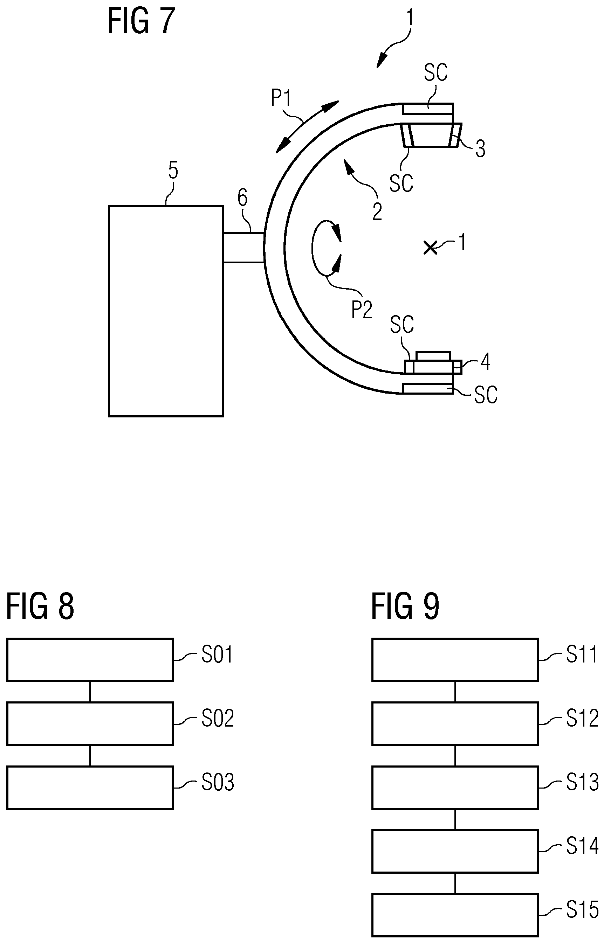

**Figur 7** zeigt eine Ansicht eines medizinischen Geräts 1 gemäß einem Ausführungsbeispiel der vorliegenden Erfindung.

Das medizinische Gerät 1 umfasst einen C-Bogen 2, an dem ein Röntgenstrahler 3 sowie ein Röntgendetektor 4 angeordnet sind. Der C-Bogen 2 ist an einem Grundgestell 5 angeordnet, mit dem er über eine entsprechende Verbindung 6 gekoppelt ist. Bei dieser Verbindung 6, die hier nur dem Grunde nach gezeigt ist, kann es sich beispielsweise um einen Mehrgelenksarm oder ein Stativ oder Ähnliches handeln. In jedem Fall ist der C-Bogen über diese Verbindung 6 im Raum bewegbar. Er ist zum einen längs seiner Bogenbahn, wie durch den Doppelpfeil P1 dargestellt ist, bewegbar, also auf einer Orbitalbahn bewegbar, so dass er um das Isozentrum I gedreht werden kann. Darüber hinaus ist er auch um eine Achse drehbar, wie durch den Pfeil P2 dargestellt. Im Fall eines Gelenkarms ist der C-Bogen 2 noch um weitere Achsen bewegbar, ebenso im Falle eines Stativs. Figur 5 zeigt eine reine Prinzipdarstellung, um grundsätzlich Bewegungsmöglichkeiten des C-Bogens darzustellen. Aus diesen Bewegungsmöglichkeiten, insbesondere einer mitunter schnell erfolgenden Orbitalbewegung längs des Pfeils P1, ergeben sich Anforderungen an eine Kollisionsüberwachung für den C-Bogen 2.

Insofern umfasst das medizinische Gerät 1 am C-Bogen 2 mehrere erfindungsgemäße Sensorcover SC, die bspw. wie mit Bezug zu den Figuren 1 bis 6 beschrieben ausgebildet sein können. Insbesondere sind der Röntgenstrahler 3 sowie der Röntgendetektor 4 und die Enden des C-Bogens 2 von einem Sensorcover SC umgeben, um die bildgebenden Komponenten zu schützen. Sowohl am C-Bogen 2, als auch an der Verbindung 6 oder dem Grundgestell 5 können weitere Sensorcover SC verbaut sein, um einen allumfassenden Kollisionsschutz für das medizinische Gerät 1 zu erzielen. Aus der Figur 5 ergibt sich, dass die Schichten DS, SS1, AS, SS2, GS, usw. des taktilen Sensorcovers SC eine dreidimensionale Freiform entsprechend der Gehäuseform des medizinischen Geräts 1 aufweisen. Durch die Fertigung des Sensorcovers SC unter Anwendung der additiven Fertigung sind i.W. beliebige Freiform-Gestaltungen des Covers denkbar. Insbesondere kann eine Anpassung der Formgebung des Sensorcovers schnell und kostengünstig erfolgen, da bspw. keine Gussformen mehr für die Herstellung der Schichten benötigt werden.

Wie bei den Sensorcovern SC, die an den Enden des C-Bogens 2 angeordnet sind erkennbar, ist das Sensorcover C vorteilhaft als ein Gehäuseteil des medizinischen Geräts 1 ausgebildet bzw. in das Gesamtgehäuse des medizinischen Geräts 1 integriert. In diesem Sinne bildet die verformbare Deckschicht DS des taktilen Sensorcovers SC die Außenseite des Gehäuses des medizinischen Geräts 1. In dieser Ausführung kann die Kollisionsschutzfunktion in das medizinische Gerät 1 implementiert werden, ohne den ,Footprint' des Geräts nachteilig zu erhöhen.

**Figur 8** zeigt eine schematische Darstellung eines erfindungsgemäßen Verfahrens gemäß einem Ausführungsbeispiel der vorliegenden Erfindung zur Herstellung eines taktilen Sensorcovers SC, bspw. wie mit Bezug zu den Figuren 1 bis 4 beschrieben.

In einem Schritt S01 wird zunächst die reversibel kompressible Abstandsschicht AS mittels einer Technik der additiven Fertigung gefertigt. Bspw. kann hier eine Netzstruktur in der gewünschten Größe mittels 3D-Druck erzeugt werden. In einem Schritt S02 werden die Sensorschichten SS1, SS2 jeweils auf den beiden gegenüberliegenden Außenseiten der Abstandsschicht AS aufgebracht. Dies kann insbesondere durch ein Aufdampfen einer Metallschicht auf die beiden Außenseiten der Abstandsschicht erfolgen. Hierbei berücksichtigt das erfindungsgemäße Verfahren, dass vor dem Aufdampfen der Metallschicht in bzw. auf der porösen Struktur der Abstandsschicht AS eine Stützschicht oder Stützstruktur an den Oberflächen der Abstandsschicht AS vorgesehen werden muss, auf die vollflächig aufgedampft werden kann. Diese Hilfskonstruktion kann dann in einem der Fertigung nachgelagerten Schritt bspw. aus der Abstandsschicht AS herausgewaschen oder herausgelöst werden. Alternativ dazu kann das Erzeugen der Sensorschichten SS1, SS2 auch ein metallisches Sintern, ein Auflegen einer vorgefertigten Metallschicht oder Leiterbahn oder eine Technik der additiven Fertigung mit einem Metallpartikel umfassenden Kunststoff umfassen. In jedem Fall wird eine Sensorschicht SS1, SS2 direkt auf die Abstandschicht AS aufgebracht und/oder an diese angefügt. Schritt S02 umfasst in Ausführungen auch das zeitgleiche Aufdampfen der Anschlusselemente AE1, AE2. In einem nächsten Schritt S03 werden die Deckschicht DS und die Grundschicht GS jeweils mittels einer Technik der additiven Fertigung auf die Außenseiten der beiden Sensorschichten SS1, SS2 aufgebracht. Bei dieser erfindungsgemäßen Vorgehensweise wird das Sensorcover SC von innen nach außen aufgebaut. In einem oder mehreren optionalen Schritten kann ferner nach Schritt S02 auch eine Schutzkapsel SK mittels einer Technik der additiven Fertigung für das Sensorcover SC hergestellt und an die Sensorschichten SS1, SS2 sowie seitlich an die Abstandsschicht AS gefügt werden. Bevorzugt werden die Schritte des Herstellungsverfahrens mit nur einer additiven Fertigungsmaschine ausgeführt. In Ausführungen kann dabei ein Teil des Sensorcovers in der Fertigungsmaschine gedreht, gewendet oder für Zwischenschritt aus der Maschine entnommen und anschließend wieder in die Fertigungsmaschine eingelegt werden, um die Fertigung verfahrensgemäß fortzusetzen. Bspw. kann die Abstandsschicht AS nach Herstellung zur metallischen Bedampfung (Schritt S02) aus der Fertigungsmaschine entnommen und anschließend für eine Herstellung und ein Fügen der Deck- und Grundschicht DS, GS, der Maschine wieder zugeführt werden. Für das Anfügen der Deckschicht DS und der Grundschicht GS könnte das Werkstück in der Maschine gewendet werden. Insbesondere die Schritte S01 und S03 sowie weitere optionale Schritte, aber in Varianten des Sensorcovers SC auch Schritt S02, können jeweils ein Verfahren eines 3D-Drucks, ein Lasersintern, ein Schmelzschichten, ein Fused Filament Verfahren oder ein Multi Jet Modeling umfassen. Die Verfahren sind an sich bekannt und werden daher nicht näher beschrieben.

Insbesondere dann, wenn die Anschlusselemente AE1, AE2 als Kontaktlöscher ausgebildet sind, können in allen Fertigungsschritten S01 bis S03 Zwischenschritte umfasst sein, die auf die Herstellung bzw. die Integration von Teilen der Anschlusselemente AE1, AE2 in die jeweiligen Schichten gerichtet sind. Dies kann auch das Fertigen von dazugehörigen Isolationsschichten und/oder Trägerschichten TS1, TS2 umfassen.

**Figur 9** zeigt eine schematische Darstellung eines erfindungsgemäßen Verfahrens zur Herstellung eines taktilen Sensorcovers SC gemäß einem anderen Ausführungsbeispiel der vorliegenden Erfindung. In dieser Ausführung wird das Sensorcover von einer der Seiten her aufgebaut. Beschrieben wird der Aufbau ausgehend von der Grundschicht GS. Analog ergäbe sich eine Schrittfolge auch für einen Aufbau ausgehend von der Deckschicht DS.

Die Wahl des Herstellungsverfahrens richtet sich bspw. nach der Formgebung des Sensorcovers SC. Sie kann aber auch basierend auf einer Materialvorgabe in Bezug auf eine der Schichten und die damit einhergehende Fertigungstechnik bzw. Fertigungsmaschine bedingt sein.

In einem ersten Schritt S11 wird also zunächst die Grundschicht GS mittels einer Technik der additiven Fertigung gefertigt. In einem Schritt S12 wird die zweite Sensorschicht SS2 auf die obere Außenseite der Grundschicht GS aufgebracht. Im Anschluss wird in Schritt S13 die reversibel kompressible Abstandsschicht AS mittels einer Technik der additiven Fertigung auf die zweite Sensorschicht SS2 aufgebracht. In einem Schritt S14 wird dann die erste Sensorschicht SS1 auf die obere Außenseite der Abstandsschicht AS aufgebracht. In Schritt S15 wird die Deckschicht DS mittels einer Technik der additiven Fertigung auf die Außenseite der ersten Sensorschicht SS 1 aufgebracht.

Auch im Rahmen dieses Verfahrens können optionale Schritte vorgesehen sein, in denen an geeigneter Stelle eingeschoben, eine Schutzkapsel SK des Sensorcovers SC gefertigt bzw. an benachbarte Schichten angefügt wird.

Weiter können optional Zwischenschritte umfasst sein, wenn die Anschlusselemente AE1, AE2 als Kontaktlöscher ausgebildet sind, wie mit Bezug zu Figur 8 beschrieben.

Zur Ausbildung der einzelnen Verfahrensschritte wird auf die Ausführungen zu Figur 8 verwiesen.

Auch im Rahmen dieses Verfahrens kann es vorgesehen sein, dass das Werkstück, insbesondere zur Fertigung der Sensorschichten SS1, SS2 aus der Fertigungsmaschine entnommen und anschließend wieder eingelegt werden kann.

Obwohl die Erfindung im Detail durch das bevorzugte Ausführungsbeispiel näher illustriert und beschrieben wurde, ist die Erfindung nicht durch dieses Ausführungsbeispiel eingeschränkt. Andere Variationen können vom Fachmann hieraus abgeleitet werden, ohne den Schutzumfang der Erfindung zu verlassen.

## Patentansprüche

1. Taktiles Sensorcover (SC) zur Detektion einer Kollision mit einem mehrschichtigen, flächigen Aufbau, umfassend
- eine reversibel deformierbare, eine Außenseite des Sensorcovers bildende Deckschicht (DS),
- eine starre, eine Innenseite des Sensorcovers bildende Grundschicht (GS),
- eine zwischen Deckschicht und Grundschicht verlaufende Sensoreinheit (SE) umfassend
o zwei Sensorschichten (SS1, SS2), und
- eine zwischen den beiden Sensorschichten angeordnete, reversibel kompressible Abstandsschicht (AS),
wobei wenigstens zwei Schichten (DS, AS, GS) als mittels einer Technik der additiven Fertigung gebildete Schichten ausgebildet sind, wobei die Sensorschichten (SS1, SS2) jeweils ein einstückig angeformtes elektrisches Anschlusselement (AE1, AE2) umfassen, wobei das Anschlusselement (AE1, AE2) zusammen mit einer der Sensorschichten (SS1, SS2) durch metallisches Aufdampfen, Metallsintern oder eine andere Technik der additiven Fertigung hergestellt ist.

2. Taktiles Sensorcover (SC) nach Anspruch 1, bei dem die wenigstens zwei mittels Technik der additiven Fertigung gebildeten Schichten (DS, AS, GS) als im Rahmen der additiven Fertigung gefügte Schichten ausgebildet sind.

3. Taktiles Sensorcover (SC) nach Anspruch 1 oder 2, wobei die Sensorschichten (SS1, SS2) leitfähig ausgebildet sind und ein metallisches Material umfassen.

4. Taktiles Sensorcover (SC) nach einem der vorherigen Ansprüche, wobei die Abstandsschicht (AS) als Textilgewirk, Netzstruktur, Gitterstruktur, Schaumstruktur ausgebildet ist oder eine Vielzahl von sich zwischen den Sensorschichten erstreckenden Stützbalken umfasst.

5. Taktiles Sensorcover (SC) nach einem der vorherigen Ansprüche, wobei die Deckschicht (DS) und die Grundschicht (GS) aus demselben Material gebildet sind und die mechanische Stabilität der beiden Schichten über die Dichte des Materials bestimmt wird.

6. Taktiles Sensorcover (SC) nach einem der vorherigen Ansprüche, wobei die Sensorschichten (SS1, SS2) jeweils wenigsten zwei Sensorsegmente (SS11, SS12, SS13, SS14) aufweisen, wobei jeweils ein erstes Sensorsegment der ersten Sensorschicht mit einem ersten Sensorsegment der zweiten Sensorschicht deckungsgleich angeordnet ist.

7. Taktiles Sensorcover (SC) nach einem der vorherigen Ansprüche, ferner umfassend eine in sich geschlossene Schutzkapsel (SK), die sich schichtartig zwischen Deckschicht (DS) und zweiter Sensorschicht (SS2), Grundschicht (GS) und erster Sensorschicht (SS1) und jeweils an allen Seiten der Sensorschichten (SS1, SS2) sowie der Abstandsschicht (AS) erstreckt.

8. Medizinisches Gerät (1) umfassend ein taktiles Sensorcover (SC) gemäß einem der Ansprüche 1 bis 7.

9. Medizinisches Gerät (1) nach Anspruch 8, wobei die Schichten des taktilen Sensorcovers (SC) eine dreidimensionale Freiform entsprechend der Gehäuseform des medizinischen Geräts aufweisen.

10. Medizinisches Gerät (1) nach Anspruch 8 oder 9, wobei die Deckschicht (DS) des taktilen Sensorcovers (SC) die Außenseite des Gehäuses des medizinischen Geräts (1) bildet.

11. Verfahren zur Herstellung eines taktilen Sensorcovers (SC) umfassend
- eine reversibel deformierbare, eine Außenseite des Sensorcovers bildende Deckschicht (DS),
- eine starre, eine Innenseite des Sensorcovers bildende Grundschicht (GS),
- eine zwischen Deckschicht und Grundschicht verlaufende Sensoreinheit (SE) umfassend
o zwei Sensorschichten (SS1, SS2), und
- eine zwischen den beiden Sensorschichten angeordnete, reversibel kompressible Abstandsschicht (AS),
wobei
- zunächst die reversibel kompressible Abstandsschicht (AS) mittels einer Technik der additiven Fertigung gefertigt wird (S01),
- die Sensorschichten (SS1, SS2) jeweils auf den beiden gegenüberliegenden Außenseiten der Abstandsschicht (AS) aufgebracht werden (S02), und
- die Deckschicht (DS) und die Grundschicht (GS) jeweils mittels einer Technik der additiven Fertigung auf die Außenseiten der beiden Sensorschichten (SS1, SS2) aufgebracht werden (S03),
wobei die Sensorschichten (SS1, SS2) jeweils ein einstückig angeformtes elektrisches Anschlusselement (AE1, AE2) umfassen, wobei das Anschlusselement (AE1, AE2) zusammen mit einer der Sensorschichten (SS1, SS2) durch metallisches Aufdampfen, Metallsintern oder eine andere Technik der additiven Fertigung hergestellt wird.

12. Verfahren zur Herstellung eines taktilen Sensorcovers (SC) umfassend
- eine reversibel deformierbare, eine Außenseite des Sensorcovers bildende Deckschicht (DS),
- eine starre, eine Innenseite des Sensorcovers bildende Grundschicht (GS),
- eine zwischen Deckschicht und Grundschicht verlaufende Sensoreinheit (SE) umfassend
o zwei Sensorschichten (SS1, SS2), und
- eine zwischen den beiden Sensorschichten angeordnete, reversibel kompressible Abstandsschicht (AS),
wobei
- zunächst die Grundschicht (S) mittels einer Technik der additiven Fertigung gefertigt wird (S11),
- die zweite Sensorschicht (SS2) auf die obere Außenseite der Grundschicht (GS) aufgebracht wird (S12),
- die reversibel kompressible Abstandsschicht (AS) mittels einer Technik der additiven Fertigung auf die zweite Sensorschicht (SS2) aufgebracht wird (S13),
- die erste Sensorschicht (SS1) auf die obere Außenseite der Abstandsschicht (AS) aufgebracht wird (S14), und
- die Deckschicht mittels einer Technik der additiven Fertigung auf die Außenseite der ersten Sensorschicht (SS1) aufgebracht werden (S15),
wobei die Sensorschichten (SS1, SS2) jeweils ein einstückig angeformtes elektrisches Anschlusselement (AE1, AE2) umfassen, wobei das Anschlusselement (AE1, AE2) zusammen mit einer der Sensorschichten (SS1, SS2) durch metallisches Aufdampfen, Metallsintern oder eine andere Technik der additiven Fertigung hergestellt wird.

13. Verfahren zur Herstellung eines taktilen Sensorcovers (SC) nach einem der Ansprüche 11 oder 12, wobei das Aufbringen der ersten und/oder der zweiten Sensorschicht (SS1, SS2) ein metallisches Sintern, ein Aufdampfen eines Metalls, ein Auflegen einer Metallschicht oder eine einer Technik der additiven Fertigung mit einem Metallpartikel umfassenden Kunststoff umfasst.

14. Verfahren zur Herstellung eines taktilen Sensorcovers (SC) nach einem der Ansprüche 11 bis 12, wobei die Technik der additiven Fertigung für jede Schicht des Sensorcovers einen 3D-Druck, ein Lasersintern, ein Schmelzschichten, ein Fused Filament Verfahren oder ein Multi Jet Modeling umfasst.

## Claims

1. Tactile sensor cover (SC) for detecting a collision with a multilayer, two-dimensional structure, comprising
- a reversibly deformable top layer (DS) forming an outer side of the sensor cover,
- a rigid base layer (GS) forming an inner side of the sensor cover,
- a sensor unit (SE) running between top layer and base layer, comprising
o two sensor layers (SS1, SS2), and
- a reversibly compressible spacer layer (AS) arranged between the two sensor layers,
wherein at least two layers (DS, AS, GS) are embodied as layers formed by means of an additive manufacturing technique, wherein the sensor layers (SS1, SS2) comprise one integrally moulded electrical connecting element (AE1, AE2) respectively, wherein the connecting element (AE1, AE2) can be produced together with one of the sensor layers (SS1, SS2) by way of metal vapour deposition, metal sintering or another additive manufacturing technique.

2. Tactile sensor cover (SC) according to claim 1, wherein at least two layers (DS, AS, GS) formed by means of the additive manufacturing technique are embodied as layers joined during the course of additive manufacturing.

3. Tactile sensor cover (SC) according to claim 1 or 2, wherein the sensor layers (SS1, SS2) are conductive and comprise a metal material.

4. Tactile sensor cover (SC) according to one of the preceding claims, wherein the spacer layer (AS) is embodied as a knitted fabric, mesh structure, grid structure, foam structure or comprises a large number of support beams extending between the sensor layers.

5. The tactile sensor cover (SC) according to one of the preceding claims, wherein the top layer (DS) and the base layer (GS) are formed from the same material and the mechanical stability of the two layers is determined via the density of the material.

6. Tactile sensor cover (SC) according to one of the preceding claims, wherein the sensor layers (SS1, SS2) have at least two sensor segments (SS11, SS12, SS13, SS14) respectively, wherein a first sensor segment respectively of the first sensor layer is congruently arranged with a first sensor segment of the second sensor layer.

7. Tactile sensor cover (SC) according to one of the preceding claims, further comprising a self-contained protective capsule (SK), which extends layer-like between top layer (DS) and second sensor layer (SS2), base layer (GS) and first sensor layer (SS1) and at all sides of the sensor layers (SS1, SS2) and the spacer layer (AS) respectively.

8. Medical device (1) comprising a tactile sensor cover (SC) according to one of claims 1 to 7.

9. Medical (1) device according to claim 8, wherein the layers of the tactile sensor cover (SC) have a three-dimensional free shape corresponding to the housing shape of the medical device.

10. Medical device (1) according to claim 8 or 9, wherein the top layer (DS) of the tactile sensor cover (SC) forms the outer side of the housing of the medical device (1).

11. Method for producing a tactile sensor cover (SC), comprising
- a reversibly deformable top layer (DS) forming an outer side of the sensor cover,
- a rigid base layer (GS) forming an inner side of the sensor cover,
- a sensor unit (SE) running between top layer and base layer, comprising
o two sensor layers (SS1, SS2), and
- a reversibly compressible spacer layer (AS) arranged between the two sensor layers,
wherein
- firstly, the reversibly compressible spacer layer (AS) is manufactured (S01) by means of an additive manufacturing technique,
- the sensor layers (SS1, SS2) are applied (S02) to the two opposing outer sides of the spacer layer (AS) respectively, and
- the top layer (DS) and the base layer (GS) are applied (S03) by means of an additive manufacturing technique respectively to the outer sides of the two sensor layers (SS1, SS2),
wherein the sensor layers (SS1, SS2) comprise one integrally moulded electrical connecting element (AE1, AE2) respectively, wherein the connecting element (AE1, AE2) can be produced together with one of the sensor layers (SS1, SS2) by way of metal vapour deposition, metal sintering or another additive manufacturing technique.

12. Method for producing a tactile sensor cover (SC) comprising
- a reversibly deformable top layer (DS) forming an outer side of the sensor cover,
- a rigid base layer (GS) forming an inner side of the sensor cover,
- a sensor unit (SE) running between top layer and base layer, comprising
o two sensor layers (SS1, SS2), and
- a reversibly compressible spacer layer (AS) arranged between the two sensor layers,
wherein
- firstly, the base layer (S) is manufactured (S11) by means of an additive manufacturing technique,
- the second sensor layer (SS2) is applied (S12) to the upper outer side of the base layer (GS),
- the reversibly compressible spacer layer (AS) is applied (S13) to the second sensor layer (SS2) by means of an additive manufacturing technique,
- the first sensor layer (SS1) is applied (S14) to the upper outer side of the spacer layer (AS), and
- the top layer is applied (S15) to the outer side of the first sensor layer (SS1) by means of an additive manufacturing technique,
wherein the sensor layers (SS1, SS2) comprise one integrally moulded electrical connecting element (AE1, AE2) respectively, wherein the connecting element (AE1, AE2) can be produced together with one of the sensor layers (SS1, SS2) by way of metal vapour deposition, metal sintering or another additive manufacturing technique.

13. Method for producing a tactile sensor cover (SC) according to one of claims 11 or 12, wherein applying the first and/or the second sensor layer (SS1, SS2) comprises metal sintering, vapour deposition of a metal, applying a metal layer or an additive manufacturing technique with a plastics material comprising metal particles.

14. Method for producing a tactile sensor cover (SC) according to one of claims 11 to 12, wherein the additive manufacturing technique for each layer of the sensor cover comprises 3D printing, laser sintering, fused deposition modelling, a fused filament method or multi jet modelling.

## Revendications

1. Recouvrement (SC) tactile de capteur pour la détection d'une collision ayant une structure stratifiée en nappe, comprenant
- une couche (DS) de recouvrement déformable réversiblement, formant une face extérieure du recouvrement de capteur,
- une couche (GS) de base rigide, formant une face intérieure du recouvrement de capteur,
- une unité (SE) de capteur s'étendant entre la couche de recouvrement et la couche de base comprenant
o deux couches (SS1, SS2) de capteur, et
- une couche (AS) de mise à distance, compressible réversiblement, disposée entre les deux couches de capteur,
dans lequel au moins deux couches (DS, AS, GS) sont constituées sous la forme de couches formées au moyen d'une technique de fabrication additive, dans lequel les couches (SS1, SS2) de capteur comprennent chacune un élément (AE1, AE2) de connexion électrique venu de matière d'une seule pièce, dans lequel l'élément (AE1, AE2) de connexion est fabriqué, ensemble avec l'une des couches (SS1, SS2) de capteur, par évaporation métallique, par frittage de métal ou par une autre technique de la fabrication additive.

2. Recouvrement (SC) tactile de capteur suivant la revendication 1, dans lequel les au moins deux couches (DS, AS, GS) formées au moyen d'une technique de la fabrication additive sont constituées sous la forme de couches jointes dans le cadre de la fabrication additive.

3. Recouvrement (SC) tactile de capteur suivant la revendication 1 ou 2, dans lequel les couches (SS1, SS2) de capteur sont constituées de manière conductrice et comprennent un matériau métallique.

4. Recouvrement (SC) tactile de capteur suivant l'une des revendications précédentes, dans lequel la couche (AS) de mise à distance est constituée sous la forme d'un tricot textile, d'une structure réticulée, d'une structure en grille, d'une structure en mousse ou d'une pluralité de supports s'étendant entre les couches de capteur.

5. Recouvrement (SC) tactile de capteur suivant l'une des revendications précédentes, dans lequel la couche (DS) de recouvrement et la couche (GS) de base sont en le même matériau et la stabilité mécanique des deux couches est déterminée par l'épaisseur du matériau.

6. Recouvrement (SC) tactile de capteur l'une des revendications précédentes, dans lequel les couches (SS1, SS2) du capteur ont chacune au moins deux segments (SS11, SS12, SS13, SS14) de capteur, dans lequel respectivement un premier segment de capteur de la première couche de capteur est disposé en coïncidence avec un premier segment de capteur de la deuxième couche de capteur.

7. Recouvrement (SC) tactile de capteur suivant l'une des revendications précédentes, comprenant en outre une capsule (SK) de protection en soi fermée, qui s'étend à la manière d'une couche entre couche (DS) de recouvrement et deuxième couche (SS2) de capteur, couche (GS) de base et première couche (SS1) de capteur et s'étend respectivement sur tous les côtés des couches (SS1, SS2) de capteur, ainsi que de la couche (AS) de mise à distance.

8. Appareil (1) médical comprenant un recouvrement (SC) tactile de capteur suivant l'une des revendications 1 à 7.

9. Appareil (1) médical suivant la revendication 8, dans lequel les couches du recouvrement (SC) tactile de capteur ont une forme libre en trois dimensions correspondant à la forme du boîtier de l'appareil médical.

10. Appareil (1) médical suivant la revendication 8 ou 9, dans lequel la couche (DS) de recouvrement du recouvrement (SC) tactile de capteur forme la face extérieure du boîtier de l'appareil (1) médical.

11. Procédé de fabrication d'un recouvrement (SC) tactile de capteur comprenant
- une couche (DS) de recouvrement déformable réversiblement, formant une face extérieure du recouvrement de capteur,
- une couche (GS) de base rigide, formant une face intérieure du recouvrement de capteur,
- une unité (SE) de capteur s'étendant entre la couche de recouvrement et la couche de base comprenant
o deux couches (SS1, SS2) de capteur, et
- une couche (AS) de mise à distance, compressible réversiblement, disposée entre les deux couches de capteur,
dans lequel
- on fabrique d'abord la couche (AS) de mise à distance, compressible réversiblement au moyen d'une technique de la fabrication additive (S01),
- on dépose les couches (SS1, SS2) de capteur respectivement sur les deux faces extérieures opposées de la couche (AS) de mise à distance (S02), et
- on dépose la couche (DS) de recouvrement et la couche (GS) de base respectivement au moyen d'une technique de la fabrication additive sur les faces extérieures des deux couches (SS1, SS2) de capteur (S03),
dans lequel les couches (SS1, SS2) de capteur comprennent chacune un élément (AE1, AE2) de connexion électrique venu de matière d'une seule pièce, dans lequel l'élément (AE1, AE2) de connexion est fabriqué, ensemble avec l'une des couches (SS1, SS2) de capteur, par évaporation métallique, par frittage de métal ou par une autre technique de la fabrication additive.

12. Procédé de fabrication d'un recouvrement (SC) tactile de capteur comprenant
- une couche (DS) de recouvremnet déformable réversiblement, formant une face extérieure du recouvrement de capteur,
- une couche (GS) de base rigide, formant une face intérieure du recouvrement de capteur,
- une unité (SE) de capteur s'étendant entre la couche de recouvrement et la couche de base comprenant
o deux couches (SS1, SS2) de capteur, et
- une couche (AS) de mise à distance, compressible réversiblement, disposée entre les deux couches de capteur,
dans lequel
- on fabrique d'abord la couche (S) de base au moyen d'une technique de la fabrication additive (S11),
- on dépose la deuxième couche (SS2) de capteur sur la face extérieure supérieure de la couche (GS) de base (S12),
- on dépose la couche (AS) de mise à distance, compressible réversiblement au moyen d'une technique de la fabrication additive sur la deuxième couche (SS2) de capteur (S13),
- on dépose la première couche (SS1) de capteur sur la face extérieure supérieure de la couche (AS) de mise à distance (S14), et
- on dépose la couche de recouvrement au moyen d'une technique de fabrication additive sur la face extérieure de la première couche (SS1) de capteur (S15),
dans lequel les couches (SS1, SS2) de capteur comprennent chacune un élément (AE1, AE2) de connexion électrique venu de matière d'une seule pièce, dans lequel l'élément (AE1, AE2) de connexion est fabriqué, ensemble avec l'une des couches (SS1, SS2) de capteur, par évaporation métallique, par frittage de métal ou par une autre technique de la fabrication additive.

13. Procédé de fabrication d'un recouvrement (SC) tactile de capteur suivant l'une des revendications 11 ou 12, dans lequel le dépôt de la première et/ou de la deuxième couches (SS1, SS2) de capteur comprend un frittage métallique, une évaporation d'un métal, une application d'une couche de métal ou une matière plastique comprenant des particules de métal d'une technique de la fabrication additive.

14. Procédé de fabrication d'un recouvrement (SC) tactile de capteur suivant l'une des revendications 11 ou 12, dans lequel la technique de la fabrication additive comprend, pour chaque couche du recouvrement de capteur, une impression en 3D, un frittage laser, un couchage par fusion, un procédé à filaments fondus ou un multi jet modeling.
